(19) Europäisches Patentamt
European Patent Office
Office européen des brevets

(11) EP 3 398 592 A1

(12) **EUROPEAN PATENT APPLICATION**
published in accordance with Art. 153(4) EPC

(43) Date of publication:
**07.11.2018 Bulletin 2018/45**

(21) Application number: **16881812.8**

(22) Date of filing: **28.12.2016**

(51) Int Cl.:
***A61K 9/70*** $^{(2006.01)}$ ***A61K 9/06*** $^{(2006.01)}$
***A61K 9/16*** $^{(2006.01)}$ ***A61K 31/445*** $^{(2006.01)}$
***A61K 47/06*** $^{(2006.01)}$ ***A61K 47/14*** $^{(2017.01)}$
***A61K 47/32*** $^{(2006.01)}$ ***A61K 47/36*** $^{(2006.01)}$

(86) International application number:
**PCT/JP2016/089052**

(87) International publication number:
**WO 2017/115838 (06.07.2017 Gazette 2017/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **28.12.2015 JP 2015255753**

(71) Applicant: **Sekisui Chemical Co., Ltd.**
**Osaka-shi, Osaka 530-8565 (JP)**

(72) Inventors:
- **AKAMINE, Takayuki**
**Mishima-gun**
**Osaka 618-0021 (JP)**
- **MATSUMOTO, Izumi**
**Mishima-gun**
**Osaka 618-0021 (JP)**
- **TONE, Saori**
**Mishima-gun**
**Osaka 618-0021 (JP)**
- **KAWAMURA, Daichi**
**Mishima-gun**
**Osaka 618-0021 (JP)**
- **ITOU, Kazushi**
**Mishima-gun**
**Osaka 618-0021 (JP)**
- **ABE, Yoshiko**
**Tokyo 103-0027 (JP)**

(74) Representative: **Ter Meer Steinmeister & Partner Patentanwälte mbB**
**Nymphenburger Straße 4**
**80335 München (DE)**

# (54) EXTERNAL PREPARATION AND METHOD FOR PRODUCING SAME

(57) An object of the present invention is to prevent a particle including a first fraction containing an active ingredient and a second fraction containing a surfactant from bleeding out from the base phase of an external preparation. The external preparation comprises a particle including a first fraction containing an active ingredient and a second fraction containing a surfactant, and a base phase containing a gelatinous liquid component.

EP 3 398 592 A1

## Description

### Technical Field

The present invention relates to external preparations, more particularly to external preparations for use as pharmaceuticals and cosmetics and a method for producing the same.

### Background Art

Recently, various external preparations of which an active ingredient of drugs and the like is brought into contact with the surface of a skin, mucous membrane, or the like, so as to be absorbed for acting on local or whole body, have been developed.

Gelatinous compositions for external application (Patent Literature 1), oily gelatinous cosmetics (Patent Literature 2), and patches using an organogel (Patent Literature 3) have been proposed as such external preparations.

A skin includes three layers consisting of an epidermis, a cutis, and a subcutaneous tissue, and the outermost epidermis layer is covered with a stratum corneum. Accordingly, application of an active ingredient to a skin surface alone cannot allow the active ingredient to efficiently pass through the skin. In particular, a hydrophilic active ingredient can hardly pass through the skin. External preparations intended for transdermal absorption are therefore required to be improved for enhancement of the skin permeability of active ingredients. As an example thereof, a method of mixing a transdermal absorption enhancer such as isopropyl myristate with an active ingredient to make a base phase, or a method of adding an active ingredient particle in a solid-in-oil state into an adhesive layer as disclosed in Patent Literature 4 has been proposed.

### Citation List

### Patent Literature

Patent Literature 1: Japanese Patent Laid-Open No. 2004-075540
Patent Literature 2: Japanese Patent Laid-Open No. 2013-227288
Patent Literature 3: Japanese Patent Laid-Open No. 2015-145429
Patent Literature 4: Japanese Patent Laid-Open No. 2014-172840


### Summary of Invention

### Technical Problem

As described above, in external preparations for transdermal absorption, using a transdermal absorption enhancer together with an active ingredient, or blending an active ingredient into external preparations to make a core-shell structure such as a particle in a solid-in-oil state, is effective to enhance the skin permeability of the active ingredient. The present inventors, however, found that blending a core-shell structured particle made of an active ingredient and a surfactant into a base phase in external preparations for transdermal absorption causes the problem of easy occurrence of a floating phenomenon of the particle from the base phase (bleed-out). In the case of external preparations for transdermal absorption for use as patch, the bleed-out allows the core-shell structure to adhere to the release liner, so that the problem of decrease in the active ingredient content in the base phase occurs. In particular, the combination of an oily liquid component and a core-shell structure tends to cause the bleed-out phenomenon. It was therefore found difficult to add a core-shell structured particle into a base phase containing oily liquid components such as liquid paraffin and isopropyl myristate.

In view of the foregoing, an object of the present invention is to provide external preparations capable of preventing the bleed-out phenomenon of a core-shell structured particle from the base phase, and to provide a method for producing external preparations capable of preventing the bleed-out phenomenon.

### Solution to Problem

Through extensive studies to solve the above problem, the present inventors found that an external preparation comprising a particle including a first fraction containing an active ingredient and a second fraction containing a surfactant, and a base phase containing a gelatinous liquid component, can suppress the bleed-out phenomenon. The present

invention has been accomplished through further trials and errors based on the finding, including the following aspects.

Aspect 1: An external preparation comprising a particle including a first fraction containing an active ingredient and a second fraction containing a surfactant, and a base phase containing a gelatinous liquid component.
Aspect 2: The external preparation according to aspect 1, wherein the particle includes a part or the whole of the surface of the first fraction covered with the second fraction.
Aspect 3: The external preparation according to aspect 1 or 2, wherein the gelatinous liquid component content is in the range of 1 to 50 wt% relative to 100 wt% of the base phase.
Aspect 4: The external preparation according to any one of aspects 1 to 3, wherein the gelatinous liquid component contains a liquid agent and a gelling agent, with a gelling agent content in the range of 1 to 30 wt% relative to 100 wt% of the liquid agent.
Aspect 5: The external preparation according to aspect 4, wherein the liquid agent is at least one oily base selected from the group consisting of hydrocarbons, alcohol carboxylic acid esters, polyalcohol fatty acid esters, and oxy acid esters.
Aspect 6: The external preparation according to aspect 4 or 5, wherein the gelling agent is at least one selected from the group consisting of polyethylene, dextrin palmitate, dextrin (palmitate/ethylhexanoate), dextrin myristate, and inulin stearate.
Aspect 7: The external preparation according to any one of aspects 1 to 6, wherein the active ingredient is hydrophilic.
Aspect 8. The external preparation according to any one of aspects 1 to 7, wherein the external preparation is a patch or an ointment.
Aspect 9: The external preparation according to aspect 8, wherein the patch contains an adhesive in the base phase.
Aspect 10: The external preparation according to aspect 9, wherein the adhesive is a rubber adhesive or an acrylic adhesive.
Aspect 11: A method for producing an external preparation comprising a step (I) of mixing a particle including a first fraction containing an active ingredient and a second fraction containing a surfactant with one or more bases, and a step of preparing a base phase by gelling the one or more bases before or after the step (I).
Aspect 12: A method for producing an external preparation comprising a step (1) of preparing a base containing a gelatinous liquid component, and a step (2) of preparing a base phase by mixing a particle including a first fraction containing an active ingredient and a second fraction containing a surfactant with the base containing a gelatinous liquid component.
Aspect 13: The method for producing an external preparation according to aspect 12, wherein the step (1) comprises mixing a liquid agent with a gelling agent.
Aspect 14: The method for producing an external preparation according to aspect 13, wherein the liquid agent is an oily base.

Advantageous Effect of Invention

According to the present invention, an external preparation for transdermal absorption capable of preventing the bleed-out phenomenon of a particle with a core-shell structure comprising an active ingredient and a surfactant from the base phase can be provided.

Description of Embodiments

In the present specification, the expressions "containing" and "comprising" include the concepts of "essentially composed of" and "only composed of".

1. Particle

A particle comprises at least two fractions including a first fraction containing an active ingredient and a second fraction containing a surfactant. The first fraction and the second fraction have only to be connected to each other (preferably through intermolecular force) to form an assembly. From the viewpoints of transdermal absorption and sustained release of the active ingredient, a part or the whole of the surface of the first fractions is preferably covered with the second fraction. For example, 30% or more, preferably 50% or more, more preferably 70% or more, still more preferably 85% or more, further more preferably 95% or more, particularly preferably 99% or more, of the surface of the first fraction is covered with the second fraction. Examples of an embodiment of the particle include a core-shell structure having a first fraction as a core part and a second fraction as a shell part enwrapping the core part.
The particle for use in the present invention has a number average particle diameter of about 1 nm to 500 nm, preferably about 1 to 100 nm, more preferably about 1 to 50 nm, still more preferably 1 to 30 nm, further more preferably

1 to 15 nm, particularly preferably 2 nm to 10 nm.

The shape of the particle is not particularly limited. With a particle diameter in the range, the active ingredient is allowed to have further improved skin permeability as well as further improved storage stability and durability, regardless of the shape. The shape of the particle may be, for example, spherical, rod-like, cubic, lenticular, and sea urchin-shaped.

In the present invention, the number average particle diameter of the particle is calculated in dynamic light scattering when the particle is dispersed in a solvent (e.g., squalane).

The moisture content of the particle is preferably 20 wt% or less, more preferably 10 wt% or less, still more preferably 5 wt% or less, further more preferably 1 wt% or less, particularly preferably substantially free of water. In other words, the particle of the present invention is different from a particle in a W/O emulsion.

In the present invention, it is preferable that the first fraction is a solid. In this case, the stability in a base to be described below is further improved. Accordingly, dispersing the particle in a base phase as oil phase allows a formulation having an S/O (solid in oil) structure to be formed.

1.1 First fraction

The first fraction comprises at least an active ingredient.

The active ingredient is not particularly limited as long as the component has a physiological activity. Preferably, the component is blended to exert its physiological activity. In this preferred embodiment, any component having a physiological activity that is not blended to exert the physiological activity from the view points of the amount blended, the blending method, etc., is not included in the active ingredient. Examples of the active ingredient include components that are blended as active ingredient into pharmaceuticals, cosmetics, etc.

As the active ingredient to be blended into pharmaceuticals, any of those for systemic action and those for local action can be used.

Specific examples of the active ingredient blended into pharmaceuticals include, but are not particularly limited thereto, therapeutic agents for dementia, antiepileptics, antidepressants, anti-Parkinson's drugs, anti-allergic drugs, anti-cancer agents, antidiabetic agents, antihypertensive agents, therapeutic agents for ED, dermatologic agents, local anesthetics, and pharmaceutically acceptable salts thereof. More specifically, examples include memantine, donepezil, rivastigmine, galantamine, nitroglycerin, lidocaine, fentanyl, male hormones, female hormones, nicotine, clomipramine, diphenhydramine, nalfurafine, metoprolol, fesoterodine, sildenafil, nalfurafine, tandospirone, beraprost sodium, taltirelin, lurasidone, nefazodone, rifaximin, benidipine, doxazosin, nicardipine, formoterol, lomerizine, amlodipine, vardenafil, octreotide, teriparatide, bucladesine, clomoglicic acid, and pharmaceutically acceptable salts thereof.

The pharmaceutically acceptable salts are not particularly limited, and any of acidic salts and basic salts can be employed. Examples of the acidic salts include inorganic acidic salts such as hydrochlorides, hydrobromides, sulfates, nitrates, and phosphate, and organic acidic salts such as acetates, propionates, tartrates, fumarates, maleates, malates, citrates, methanesulfonates, benzenesulfonates, and p-toluenesulfonates. Examples of the basic salts include salts of alkali metals such as sodium and potassium, and alkaline earth metal salts such as calcium salts and magnesium salts. Examples of the salt of the active ingredient include memantine hydrochloride, donepezil hydrochloride, rivastigmine tartrate, galantamine hydrobromide, clomipramine hydrochloride, diphenhydramine hydrochloride, nalfurafine hydrochloride, metoprolol tartrate, fesoterodine fumarate, sildenafil hydrochloride hydrate, nalfurafine hydrochloride, tandospirone citrate, beraprost sodium, lurasidone hydrochloride, nefazodone hydrochloride, benidipine hydrochloride, doxazosin mesylate, nicardipine hydrochloride, formoterol fumarate, lomerizine hydrochloride, and amlodipine besylate.

The active ingredient to be blended into cosmetics is not particularly limited as long as skin permeation is required, and examples thereof include vitamin components such as vitamin C and Vitamin E, moisturizing components such as hyaluronic acid, ceramide, and collagen, whitening components such as tranexamic acid and arbutin, hair growth components such as minoxidil, cosmetic components such as FGF (fibroblast growth factor), EGF (epidermal growth factor), and the salts and derivatives thereof.

Preferably, the active ingredient is hydrophilic.

In the case of an active ingredient made of hydrophilic chemical, the active ingredient has a molecular weight of typically 10000 or less, and an octanol/water partition coefficient of -6 to 6, though not particularly limited thereto.

In the above description, the molecular weight is more preferably 5000 or less, still more preferably 2000 or less. The lower limit of molecular weight is typically 50, though not particularly limited.

In the above description, the octanol/water partition coefficient is more preferably -6 to 5, still more preferably -3 to 4.

In the present invention, the octanol/water partition coefficient is obtained as follows. After the chemical is added into a flask containing octanol and an aqueous buffer at pH 7, the mixture is shaken. Based on the chemical concentration in each phase, the octanol/water partition coefficient is calculated by the following equation. Octanol/water partition coefficient = Log10 (concentration in octanol phase/concentration in aqueous phase)

The amount of the active ingredient contained in the particle depends on the type of the active ingredient, and

the weight of raw material added may be, for example, 0.1 to 30 wt% (based on the total weight of all the raw materials contained in the particle).

The first fraction may contain two or more active ingredients on an as needed basis.

The first fraction may further contain at least one other component in addition to the active ingredient. Examples of the other component include stabilizers, transdermal absorption enhancer, skin irritation reducing agents and antiseptics, though not limited thereto.

Stabilizers have a function for stabilizing the structure of a particle, preventing the unintentional collapse of particle structure in an early stage, and ensuring the sustained release effect of the active ingredient.

Specific examples of the stabilizer include polysaccharides, proteins, and hydrophilic polymeric materials, though not particularly limited. One or two or more stabilizers may be contained. The stabilizer content in the first fraction may be appropriately set depending on the type, and the weight ratio between the active ingredient and the stabilizer in the formulation may be set, for example, at 1:0.1 to 1:10.

Specific examples of the transdermal absorption enhancer include higher alcohols, N-acyl sarcosine and salts thereof, higher monocarboxylic acids, higher monocarboxylic acid esters, aromatic monoterpene fatty acid esters, dicarboxylic acids having 2 to 10 carbon atoms and salts thereof, polyoxyethylene alkyl ether phosphate and salts thereof, lactic acid, lactates, and citric acid, though not particularly limited thereto. One or two or more transdermal absorption enhancers may be contained. The transdermal absorption enhancer content in the first fraction may be appropriately set depending on the type, and the weight ratio between the active ingredient and the transdermal absorption enhancer in the formulation may be set, for example, at 1:0.01 to 1:50.

Specific examples of the skin irritation reducing agent include hydroquinone glycosides, pantethine, tranexamic acid, lecithin, titanium oxide, aluminum hydroxide, sodium nitrite, sodium hydrogen nitrite, soy lecithin, methionine, glycyrrhetinic acid, BHT, BHA, vitamin E and derivatives thereof, vitamin C and derivatives thereof, benzotriazole, propyl gallate, and mercaptobenzimidazole, though not particularly limited thereto. One or two or more skin irritation reducing agents may be contained. The skin irritation reducing agent content in the first fraction may be appropriately set depending on the type, and may be set, for example, at 0.1 wt% to 50 wt% in the formulation.

Specific examples of the antiseptic include methyl parahydroxybenzoate, propyl parahydroxybenzoate, phenoxyethanol and thymol, though not particularly limited thereto. The antiseptic content in the first fraction may be appropriately set depending on the type, and may be set, for example, at 0.01 wt% to 10 wt% in the formulation. One or two or more antiseptics may be contained.

1.2 Second fraction

The second fraction comprises at least a surfactant.

A surfactant having a weight average HLB (abbreviation of Hydrophile Lypophile Balance) value of preferably 10 or less, more preferably 5 or less, still more preferably 3 or less, may be used.

The HLB value in the present invention is an indicator of whether an emulsifier is hydrophilic or lipophilic, taking a value of 0 to 20. The smaller HLB value indicates higher lipophilic. The HLB value is calculated from the following Griffin equation in the present invention.

$$\text{HLB value} = 20 \times \{(\text{molecular weight of hydrophilic moiety})/(\text{total molecular weight})\}$$

The weight average HLB value is calculated as follows.

For example, when surfactant materials having HLB values A, B, and C, with weights of x, y, and z, respectively, are added for particle synthesis, the calculation formula for the weight average is represented by: $(xA + yB + zC)/(x + y + z)$.

The surfactant is not particularly limited, and may be appropriately selected depending on the application. For example, it may be selected widely from those that can be used in pharmaceuticals and cosmetics. A plurality of surfactants may be used in combination.

The surfactant may be any of nonionic surfactants, anionic surfactants, cationic surfactants and amphoteric surfactants.

Examples of the nonionic surfactant include fatty acid esters, fatty alcohol ethoxylates, polyoxyethylene alkyl phenyl ethers, alkyl glycosides and fatty acid alkanolamides, and polyoxyethylene castor oil and hardened castor oil, though not particularly limited thereto.

The fatty acid ester is not particularly limited, and sugar fatty acid esters are preferred. Specific examples include esters of a fatty acid such as erucic acid, oleic acid, lauric acid, stearic acid and behenic acid, and sucrose.

Examples of the other fatty acid ester include esters of at least one of glycerin, polyglycerin, polyoxyethylene

glycerin, sorbitan and polyoxyethylene sorbitol, and a fatty acid, though not particularly limited.

Examples of the anionic surfactant include alkyl sulfates, polyoxyethylene alkyl ether sulfate, alkyl benzene sulfonates, fatty acid salts and phosphates.

Examples of the cationic surfactant include alkyltrimethylammonium salts, dialkyldimethylammonium salts, alkyldimethylbenzylammonium salts and amine salts.

Examples of the amphoteric surfactant include alkylamino fatty acid salts, alkyl betaines and alkyl amine oxides.

As the surfactant, sucrose fatty acid esters, glycerin fatty acid esters, polyoxyethylene glycerin fatty acid esters, sorbitan fatty acid esters, polyoxyethylene sorbit fatty acid esters, polyoxyethylene castor oil and hardened castor oil are particularly preferably used.

The surfactants may include, but are not particularly limited to, those having a hydrocarbon chain (alkyl chain, alkenyl chain, alkynyl chain, etc.). The hydrocarbon chain length is not particularly limited, and may be selected widely from those having 8 to 30 carbon atoms in the main chain. The particularly preferable length is 10 to 24. More preferably, the surfactant is at least one selected from the group consisting of sucrose fatty acid esters, glycerin fatty acid esters, polyoxyethylene glycerin fatty acid esters, sorbitan fatty acid esters, polyoxyethylene sorbit fatty acid esters, polyoxyethylene castor oil and hardened castor oil.

Since a surfactant having a melting point of 50°C or lower in particular tends to cause the bleed-out phenomenon, the present invention can be applied to the case where a surfactant having a melting point of preferably 50°C or lower, more preferably 40°C or lower, is used.

In the case using only a surfactant having a hydrocarbon chain, or in the case using a surfactant having a hydrocarbon chain in combination with other surfactants, the particle of the present invention has more excellent absorption sustainability when having a weight ratio between the active ingredient and the total hydrocarbon chains contained in the surfactants of 1:1 to 1:70. In this regard, the weight ratio is preferably 1:2 to 1:70 or 1:2 to 1:50, more preferably 1:3 to 1:30, still more preferably 1:5 to 1:20.

The second fraction may further contain at least one other component in addition to the surfactant. Examples of the other component may include, but are not particularly limited to, skin irritation reducing agents, analgesics, transdermal absorption enhancers, stabilizers, and antiseptics.

Specific examples of the skin irritation reducing agent may include, but are not particularly limited to, hydroquinone glycosides, pantethine, tranexamic acid, lecithin, titanium oxide, aluminum hydroxide, sodium nitrite, sodium hydrogen nitrite, soy lecithin, methionine, glycyrrhetinic acid, BHT, BHA, vitamin E and derivatives thereof, vitamin C and derivatives thereof, benzotriazole, propyl gallate, and mercaptobenzimidazole. One or two or more skin irritation reducing agents may be contained. The content of skin irritation reducing agents in the second fraction may be appropriately set depending on the type, and may be set at, for example, 0.1 wt% to 50 wt% in the formulation.

Specific examples of the analgesic may include, but are not particularly limited to, a local anesthetic such as procaine, tetracaine, lidocaine, dibucaine and prilocaine, and salts thereof. One or two or more analgesics may be contained. The content of analgesic in the second fraction may be appropriately set depending on the type, and may be set at, for example, 0.1 wt% to 30 wt% in the formulation.

Specific examples of the transdermal absorption enhancer may include, but are not particularly limited to, higher alcohols, N-acyl sarcosine and salts thereof, higher monocarboxylic acids, higher monocarboxylic acid esters, aromatic monoterpene fatty acid esters, dicarboxylic acids having 2 to 10 carbon atoms and salts thereof, polyoxyethylene alkyl ether phosphates and salts thereof, lactic acid, lactates and citric acid. One or two or more transdermal absorption enhancers may be contained. The content of transdermal absorption enhancers in the second fraction may be appropriately set depending on the type, and may be set, for example, at 0.1 wt% to 30 wt% in the formulation.

Stabilizers have a function for stabilizing the core-shell structure, preventing the unintentional collapse of the core-shell structure in an early stage, and ensuring the sustained release effect of the active ingredient.

Specific examples of the stabilizer may include, but are not particularly limited to, fatty acids and salts thereof, p-hydroxybenzoic acid esters such as methyl paraben and propyl paraben, alcohols such as chlorobutanol, benzyl alcohol, and phenylethyl alcohol, thimerosal, acetic anhydride, sorbic acid, sodium hydrogen sulfite, L-ascorbic acid, sodium ascorbate, butylhydroxyanisole, butylhydroxy toluene, propyl gallate, tocopherol acetate, dl-$\alpha$-tocopherol, proteins and polysaccharides. One or two or more stabilizers may be contained. The content of stabilizers in the second fraction may be appropriately set depending on the type, and the weight ratio between sucrose fatty acid ester and stabilizers in the formulation may be set, for example, at 1:0.01 to 1:50.

Specific examples of the antiseptic may include, but are not particularly limited to, methyl parahydroxybenzoate, propyl parahydroxybenzoate, phenoxyethanol and thymol. One or two or more antiseptics may be contained. The content of antiseptics in the second fraction may be appropriately set depending on the type, and may be set, for example, at 0.01 wt% to 10 wt% in the formulation.

2. External preparation

The external preparations of the present invention are not particularly limited as long as, for example, the dosage form thereof is applicable to an epidermis surface such as the skins and the mucous membranes of a body so as to be rubbed into, sprayed on, applied to, or stuck on the epidermis surface. In addition to those whose active ingredient acts locally on the applied region, the external preparations of the present invention include those for transdermal absorption whose active ingredient is absorbed through the skin to act at the periphery of the applied region or around the whole body. More specifically, examples thereof include patches such as plasters, tapes (reservoir-type, matrix-type, etc.) such as emplastrums, cataplasms, transdermal patches, and microneedles, ointments such as oily ointments, liniments, liquids for external use such as lotions, aerosols for external use, sprays such as pump sprays, creams, gels, eye drops, eye ointments, nasal drops, suppositories, semisolids for application to rectum, and enema agents. In particular, patches, ointments, eye drops, nasal drops are preferred.

Depending on the type of active ingredient, the external preparations of the present invention may be used in varieties of applications including external pharmaceuticals such as external preparations for transdermal absorption, skin external preparations, eye drops, nasal drops, suppositories, and oral cavity agent, quasi drugs for external use, medicated cosmetics and cosmetics.

Intended use of the external preparations of the present invention includes medical treatment, prevention of diseases or symptoms, symptom relief, and/or cosmetic treatment, being appropriately selected depending on the type of the active ingredient.

The external preparations of the present invention provide a typical sustained release of 1 day to 1 week, though not particularly limited thereto. In a preferred embodiment, the external preparations are applied once per day to once per week.

The external preparations of the present invention contain at least the particle described in the section 1.

The content of the particle in the external preparations is, preferably 5 to 50 wt%, more preferably 5 to 40 wt%, still more preferably 10 to 30 wt%, relative to 100 wt% of the base phase of the external preparations, though not particularly limited thereto.

The weight ratio between the active ingredient and the surfactant (active ingredient weight/surfactant weight) in the particle may be appropriately set in a range where the effect of the present invention can be attained, for example, 1:3 to 1:100. In preparation of external preparations having more excellent absorption, the weight ratio is set at preferably 1:5 to 1:100, more preferably 1:10 to 1:50, still more preferably 1:15 to 1:50.

In the external preparation of the present invention, the water content in the base phase is preferably 20 wt% or less, more preferably substantially free of water. Consequently, the shape retention ability of the particle can be further enhanced, and not only the leakage of the active ingredient from the particle but also the crystallization of the active ingredient can be further suppressed, so that the transdermal absorption can be further enhanced. In this respect, the base phase of the present invention is preferably used in agents of which water content is adjusted to 20 wt% or less (more preferably in agents containing substantially no water), such as tapes, transdermal patches, plasters, ointments, gels, eye drops, and nasal drops.

The external preparations of the present invention contain a phase (base phase) containing the particle and one or more bases. In this case, the particle is dispersed or dissolved in the base phase. Preferably, the base phase contains a gelling agent. In the case of an external preparation as patch, an adhesive and, if necessary, a tackifier or a polymerization initiator can be added to the base phase. In addition, the base phase may contain other additive components corresponding to the dosage form and intended use of the external preparation. Examples of the other additive components include plasticizers, excipients, coloring agents, lubricants, binders, emulsifiers, thickening agents, wetting agents, stabilizers, preservatives, solvents, solubilizing agents, suspending agents, buffering agents, pH adjusting agents, antioxidants, transdermal absorption enhancer, irritation reducing agents, antiseptics, chelating agents, and dispersing agents.

The base is not particularly limited and may be widely selected from those that can be used as pharmaceuticals (external preparations, in particular) and cosmetics.

The base may be appropriately selected from those suitable for dispersing or dissolving a particle and other components corresponding to the intended use, and is not particularly limited.

A plurality of bases may be used in combination.

As described above, preferably the particle used in the present invention includes a first fraction of solid. In the case of a base phase of oil, such a particle is dispersed in the base phase as oil phase, so that an S/O (solid in oil) formulation can be formed. The S/O formulation can be obtained, for example, by dispersing a particle obtained by a production method including the step of drying W/O emulsion described below in an oil phase.

Examples of the base include, but are not particularly limited to, oily bases and aqueous bases. Examples of the oily base include vegetable oils, animal oils, neutral lipids, synthetic oils, sterol derivatives, waxes, hydrocarbons, monoalcohol carboxylic acid esters, oxy acid esters, polyhydric alcohol fatty acid esters, silicones, higher alcohols, higher

fatty acids, and fluorine oils. Examples of the aqueous base include water and (poly)alcohols. Oily bases are preferred.

Examples of the vegetable oils include, but are not particularly limited to, soybean oil, sesame oil, olive oil, coconut oil, palm oil, rice oil, cottonseed oil, sunflower oil, rice bran oil, cacao butter, corn oil, safflower oil and rapeseed oil.

Examples of the animal oil include, but are not particularly limited to, mink oil, turtle oil, fish oil, beef oil, horse oil, lard, and shark squalane.

Examples of the neutral lipid include, but are not particularly limited to, triolein, trilinolein, trimyristin, tristearin and triarachidonin.

Examples of the synthetic oil include, but are not particularly limited to, phospholipids and azone.

Examples of the sterol derivative include, but are not particularly limited to, dihydrocholesterol, lanosterol, dihydrolanosterol, phytosterol, and cholic acid and cholesteryl linoleate.

Examples of the wax include candelilla wax, carnauba wax, rice wax, Japan wax, beeswax, montan wax, ozokerite, ceresin, paraffin wax, microcrystalline wax, petrolatum, Fischer-Tropsch wax, polyethylene wax and ethylene-propylene copolymers.

Examples of the hydrocarbon include liquid paraffin (mineral oil), heavy liquid isoparaffin, light liquid isoparaffin, $\alpha$-olefin oligomers, polyisobutene, hydrogenated polyisobutene, polybutene, squalane, olive-derived squalane, squalene, vaseline, and solid paraffin.

Examples of the alcohol carboxylic acid esters include octyldodecyl myristate, hexyldecyl myristate, octyldodecyl isostearate, cetyl palmitate, octyldodecyl palmitate, cetyl octanoate, hexyldecyl octanoate, isotridecyl isononanoate, isononyl isononanoate, octyl isononanoate, isotridecyl isononanoate, isodecyl neopentanoate, isotridecyl neopentanoate, isostearyl neopentanoate, octyldodecyl neodecanoate, oleyl oleate, octyldodecyl oleate, octyldodecyl, octyldodecyl lanolate, hexyldecyl dimethyloctanoate, octyldodecyl erucate, hydrogenated castor oil isostearate, ethyl oleate, avocado oil fatty acid ethyl, isopropyl myristate, isopropyl palmitate, octyl palmitate, isopropyl isostearate, isopropyl lanolate, diethyl sebacate, diisopropyl sebacate, dioctyl sebacate, diisopropyl adipate, dibutyloctyl sebacate, diisobutyl adipate, dioctyl succinate, and triethyl citrate.

Examples of the oxy acid esters include cetyl lactate, diisostearyl malate, and hydrogenated castor oil monoisostearate.

Examples of the poly alcohol fatty acid esters include glyceryl trioctanoate, glyceryl trioleate, glyceryl triisostearate, glyceryl diisostearate, glyceryl tri(caprylate/caprate), glyceryl tri(caprylate/caprate/myristate/stearate), hydrogenated rosin triglyceride (hydrogenated ester gum), rosin triglyceride (ester gum), glyceryl(behenate/eicosadioate), trimethylolpropane trioctanoate, trimethylolpropane triisostearate, neopentyl glycol dioctanoate, neopentyl glycol dicaprate, 2-butyl-2-ethyl-1,3-propanediol dioctanoate, propylene glycol dioleate, pentaerythrityl tetraoctanoate, hydrogenated rosin pentaerythrityl, ditrimethylolpropane triethylhexanoate, ditrimethylolpropane(isostearate/sebacate), pentaerythrityl triethylhexanoate, dipentaerythrityl(hydroxystearate/stearate/rosinate), diglyceryl diisostearate, polyglyceryl tetraisostearate, polyglyceryl-10 nonaisostearate, polyglyceryl-8 deca(erucate/isostearate/ricinoleate), diglyceryl(hexyldecanoate/sebacate) oligoester, glycol distearate (ethylene glycol distearate), 3-methyl-1,5-pentanediol dineopentanoate, and 2,4-diethyl-1,5-pentadiol dineopentanoate.

Examples of the silicones include dimethicone (dimethyl polysiloxane), highly polymerized dimethicone (highly polymerized dimethylpolysiloxane), cyclomethicone (cyclic dimethylsiloxane, decamethylcyclopentasiloxane), phenyl trimethicone, diphenyl dimethicone, phenyl dimethicone, stearoxy propyl dimethylamine, (aminoethyl aminopropyl methicone/dimethicone) copolymers, dimethiconol, dimethiconol crosspolymers, silicone resins, silicone rubber, amino-modified silicones such as aminopropyl dimethicone and amodimethicone, cation-modified silicones, polyether-modified silicones such as dimethicone copolyol, polyglycerin-modified silicone, sucrose-modified silicones, carboxylic acid-modified silicones, phosphoric acid-modified silicone, sulfuric acid-modified silicones, alkyl-modified silicone, fatty acid-modified silicones, alkyl ether-modified silicones, amino acid-modified silicones, peptide-modified silicones, fluorine-modified silicones, cation-modified or polyether-modified silicones, amino-modified or polyether-modified silicones, alkyl-modified or polyether modified silicones, and polysiloxane/oxyalkylene copolymers.

Examples of the higher alcohols include cetanol, myristyl alcohol, oleyl alcohol, lauryl alcohol, cetostearyl alcohol, stearyl alcohol, arachyl alcohol, behenyl alcohol, jojoba alcohol, chimyl alcohol, selachyl alcohol, batyl alcohol, hexyl decanol, isostearyl alcohol, 2-octyldodecanol and dimer diols.

Examples of the higher fatty acids include lauric acid, myristic acid, palmitic acid, stearic acid, isostearic acid, behenic acid, undecylenic acid, 12-hydroxystearic acid, palmitoleic acid, oleic acid, linoleic acid, linolenic acid, erucic acid, docosahexaenoic acid, eicosapentaenoic acid, isohexadecanoic acid, anteisoheneicosanic acid, long-chain branched fatty acids, dimer acids and hydrogenated dimer acids.

Examples of the fluorine oil include perfluorodecane, perfluorooctane, and perfluoro polyether.

Examples of the (poly)alcohol include ethanol, isopropanol, glycerin, propylene glycol, 1,3-butylene glycol, and polyethylene glycol.

Examples of the other base include, but are not particularly limited to, those suitable for dosage forms including patches (plasters, tapes (reservoir-type, matrix-type, etc.) such as emplastrums, cataplasms, transdermal patches,

microneedles, etc.), ointments, liquids for external use (liniments, lotions, etc.), sprays (aerosols for external use, pump sprays, etc.) creams, gels, eye drops, eye ointments, nasal drops, suppositories, semisolids for application to rectum, and enema agents.

Among the bases for use in the present invention, those in a liquid state at room temperature (e.g., in the range of 15 to 35°C) at least prior to addition to the base phase are referred to as liquid agents. Although the liquid agent may be an oily base or an aqueous base, an oily base is preferred. Examples thereof include at least one selected from the group consisting of hydrocarbons (liquid paraffin, heavy liquid isoparaffin, light liquid isoparaffin, $\alpha$-olefin oligomers, squalane, olive-derived squalane, squalene, etc.), alcohol carboxylic acid esters (isopropyl myristate, isopropyl palmitate, isopropyl isostearate, isononyl isononanoate, isotridecyl isononanoate, etc.), polyalcohol fatty acid esters (glyceryl tri(caprylate/caprate), glyceryl trioctanoate, neopentyl glycol dioctanoate, etc.), and oxy acid esters (hydrogenated castor oil monoisostearate, etc.).

The content of the liquid agent may be appropriately set depending on the type of external preparation, preferably at 5 to 80 wt%, more preferably at 10 to 60 wt%.

In the external preparation of the present invention, use of a gelatinous liquid component made by gelling the liquid agent and/or a gelatinous base phase made by gelling the base phase after preparation is preferred.

Gelling referred to herein is formation of a three-dimensional network structure through partial cross-linking of molecules in a liquid comprising low molecular weight molecules or high molecular weight molecules or consisting of low molecular weight molecules or high molecular weight molecules. Although gelling may be performed by any of physical cross-linking and chemical cross-linking, physical cross-linking allowing the cross-linking to be reversible is preferred than chemical cross-linking allowing the cross-linking to be strong and semi-permanent, in order to enhance the absorption of an active ingredient.

Examples of the physical cross-linking method include, but are not particularly limited to, a cross-linking method by adding a gelling agent to cause hydrogen bonds in the molecules in the liquid, a cross-linking method by heating a molecule-containing liquid to form hydrophobic aggregations due to hydrophobic interaction between the molecules, and a microcrystalline cross-linking method. Physical cross-linking using a gelling agent or physical cross-linking by heating is preferred, and physical cross-linking by a gelling agent is more preferred. Alternatively, a plurality of physical cross-linking methods may be combined.

On the other hand, examples of the chemical cross-linking method include polycondensation and radical polymerization.

The gelling agent is not limited as long as gelling of a base phase or a liquid agent is achieved, and examples thereof include esters of one or more fatty acids and one polysaccharide. Examples of the fatty acid include fatty acids having preferably 5 to 26 carbon atoms, more preferably 6 to 18 carbon atoms. Preferred examples of the polysaccharide include dextrin, inulin, and sucrose. Specific examples of the ester include at least one selected from the group consisting of dextrin palmitate, dextrin palmitate/ethylhexanoate, dextrin myristate, and inulin stearate. Examples of the agent for gelling hydrocarbons such as liquid paraffin include a low molecular weight polyethylene. Examples of the polyethylene include a polyethylene having a molecular weight of preferably 50000 or less, more preferably 25000 or less.

The content of gelling agent relative to 100 wt% of a base phase is 0.1 to 10 wt%, preferably 0.2 to 8 wt%. The content of gelling agent relative to 100 wt% of a liquid agent is 1 to 30 wt%, preferably 2 to 20 wt%, more preferably 2.5 to 10 wt%.

A gelatinous liquid component in an amount corresponding to the content of the gelatinous liquid component in the base phase may be added. The content of the gelatinous liquid component is preferably 5 to 50 wt%, more preferably 10 to 40 wt%, relative to 100 wt% of the base phase. The viscosity of the gelatinous liquid component is more preferably 1000 mPa·s or more, particularly preferably 10000 mPa·s or more. The upper limit of the viscosity is not particularly limited.

For the external preparation of the present invention as patches, an adhesive may be used in the base phase, and in addition thereto, a tackifier may be used on an as needed basis.

Examples of the adhesive are not particularly limited as long as the external preparation can be stuck to the skin or the like, and for example, rubber adhesives, acrylic adhesives, vinyl adhesives, vinylpyrrolidone adhesives, and silicone adhesives may be used. Rubber adhesives, acrylic adhesives, vinyl adhesives, or vinylpyrrolidone adhesives are preferred, and rubber adhesives are more preferred.

The rubber adhesive is not particularly limited, and can be appropriately selected from among those commonly used corresponding to the specific application such as pharmaceuticals and cosmetics.

As the rubber adhesive, for example, those having a solubility parameter (SP value) calculated from Okitsu's equation of 8.7 or less may be used. In terms of transdermal absorption amount of an active ingredient, thermoplastic elastomers having an SP value of 7 to 8.7 may be used.

The SP value is an index indicating the hydrophilicity, and Okitsu's equation is used in the method for calculating $\Delta F$ in the solubility parameter represented by the following formula, (Reference: Toshinao Okitsu, Journal of the Adhesion Society of Japan, vol. 29, No. 5, 204-211 (1993)).

$$\Delta\delta = \Delta F/\Delta V$$

wherein, $\delta$ represents solubility parameter, F represents molar attraction constant, and V represents molar volume.

Specific examples of the rubber adhesive include rubber-based ones such as a styrene-isoprene-styrene block copolymer (SIS), a styrene-butadiene-styrene block copolymer (SBS), a styrene-ethylene/butylene-styrene block copolymer (SEBS), polyisobutylene (PIB) and isoprene rubber (IR), silicone-based ones such as silicone rubber, and urethane-based ones. One of the rubber adhesives may be used singly or a plurality thereof may be used in combination.

The content of the rubber adhesive is not particularly limited, and can be appropriately set. For example, the content of the rubber adhesive is preferably 1 to 50 wt%, more preferably 5 to 30 wt%, still more preferably 10 to 25 wt%, relative to 100 wt% of the base phase.

In use of a rubber adhesive, a tackifier may be further added to the base phase. Examples of the tackifier include alicyclic saturated hydrocarbon resins, terpene resins, terpene phenol resins, hydrogenated terpene resins, hydrogenated terpene phenol resins, and rosin derivatives. In particular, alicyclic saturated hydrocarbon resins and rosin derivatives are preferred.

The mixing ratio between a rubber adhesive and a tackifier is preferably in a range of 0.1 to 2 parts by weight, more preferably in a range of 0.2 to 1 part by weight, relative to 1 part by weight of the rubber adhesive.

The rubber adhesive may contain a plasticizer such as a hydrocarbon liquid paraffin. In this case also, use of the gelling agent in combination allows the bleed-out phenomenon caused by the plasticizer to be more effectively suppressed. When a hydrocarbon such as liquid paraffin is used as plasticizer, examples of the agent for gelling include polyethylene. Preferably the polyethylene has a molecular weight of 50000 or less, more preferably 25000 or less.

Examples of the acrylic adhesive, the vinyl adhesive, or the vinylpyrrolidone adhesive include a polymer of at least one monomer selected from the group consisting of compounds represented by the following general formulas (1) and (2), respectively, and a vinylpyrrolidone compound.

[Formula 1]

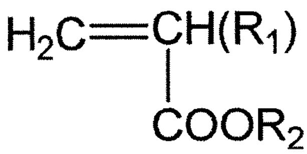

(1)

wherein, R1 represents a hydrogen atom or a methyl group, and R2 represents an alkyl group having 1 to 5 carbon atoms.

[Formula 2]

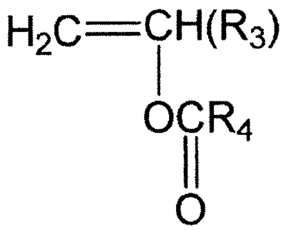

(2)

wherein, R3 represents a hydrogen atom or a methyl group, and R4 represents an alkyl group having 1 to 5 carbon atoms.

The compounds represented by the general formulas (1) and (2), respectively, and the vinylpyrrolidone compound are monomers having an SP value of preferably 8.7 to 12.

Specific examples of the compound represented by the general formula (1) include methyl acrylate (<SP value> = 9.0), ethyl acrylate (<SP value> = 8.9), methyl methacrylate (<SP value> = 8.8), and ethyl methacrylate (<SP value> = 8.7)

Specific examples of the compound represented by the general formula (2) include vinyl acetate (<SP value> = 9.0), vinyl propionate (<SP value> = 8.9), and vinyl butyrate (<SP value> = 8.8).

Specific examples of the vinylpyrrolidone compound include N-vinylpyrrolidone (<SP value> = 11.1), and N-vinylpiperidone (<SP value> = 10.6)

One of the acrylic adhesive, the vinyl adhesive, and the vinylpyrrolidone adhesive may be used singly or a plurality thereof may be used in combination.

A commonly known method may be used for polymerizing at least one monomer selected from the group consisting of the compound represented by the general formula (1), the compound represented by the general formula (2), and a vinylpyrrolidone compound. For example, a method of using a polymerization initiator may be used, and examples of the polymerization initiator include azobis-based polymerization initiators such as 2,2'-azobisisobutyronitrile (AIBN), 1,1'-azobis(cyclohexane-1-carbonitrile), 2,2'-azobis-(2,4'-dimethyl valeronitrile), and organic peroxides such as benzoyl peroxide (BPO), lauroyl peroxide (LPO), and di-tert-butyl peroxide. In particular, lauroyl peroxide or benzoyl peroxide is preferred. Two or more of the polymerization initiators may be used in combination.

The content of the acrylic adhesive, the vinyl adhesive, or the vinylpyrrolidone adhesive is not particularly limited, and may be appropriately set. For example, the content of the acrylic adhesive may be set at preferably 10 to 90 wt%, more preferably 20 to 80 wt%, still more preferably 30 to 70 wt%, relative to 100 wt% of the base phase.

Even in use of the acrylic adhesive, the vinyl adhesives, or the vinylpyrrolidone adhesive, a tackifier such as an alicyclic saturated hydrocarbon resin and a rosin derivative may be used. The mixing ratio between the acrylic adhesive and the tackifier is preferably in a range of 0.1 to 20 parts by weight, more preferably in the range of 1 to 5 parts by weight, of the tackifier, relative to 100 parts by weight of the acrylic adhesive.

Specific examples of the other additive components (plasticizers, excipients, coloring agents, lubricants, binders, emulsifiers, thickening agents, wetting agents, stabilizers, preservatives, solvents, solubilizers, suspending agents, buffering agents, pH adjusting agents, antioxidants, transdermal absorption enhancers, irritation reducing agents, antiseptics, chelating agents, dispersing agents, etc.), are not particularly limited, and examples thereof include components generally acceptable for addition to external preparations such as pharmaceuticals and cosmetics. Also, the content of each component of the other additives is not particularly limited as long as an intended external preparation can be prepared, and the component in an amount ranging from 0.00001 to 10 wt% relative to 100 wt% of the base phase may be appropriately added.

Further, in the external preparation of the present invention, the base phase in a state of containing the particle may be further dispersed in another component. In this case, the external preparation of the present invention is provided by mixing and dispersing or emulsifying the base phase into a component in which the base phase or components contained in the base phase are not completely dissolved. The selection may be appropriately performed corresponding to the dosage form without specific limitations. For example, in order to provide patches (plasters, tapes (reservoir-type, matrix-type, etc.) such as emplastrums, cataplasms, transdermal patches, microneedles, etc.), ointments, liquids for external use (liniments, lotions, etc.), sprays (aerosols for external use, pump sprays, etc.), creams, gels, eye drops, eye ointments, nasal drops, suppositories, semisolids for application to rectum, and enema agents, the base phase may be mixed and dispersed or emulsified into the base for use in each dosage form.

## 3. Method for producing a particle and external preparation

### 3.1 Method for producing a particle

The method for producing the particle of the present invention described in the section 1 is not particularly limited, and may be produced, for example, by a method including the step of drying a W/O emulsion containing an active ingredient in the aqueous phase.

A W/O emulsion containing an active ingredient in the aqueous phase, may be obtained, for example, by mixing an aqueous solvent (e.g., water, buffer aqueous solution, etc.) containing the active ingredient with an oily solvent (e.g. cyclohexane, hexane, toluene, etc.) containing a surfactant. The aqueous solvent containing an active ingredient may contain additive components such as stabilizers, transdermal absorption enhancers, skin irritation reducing agents, etc., in addition to the active ingredient on an as needed basis. The oily solvent containing a surfactant may contain additive components such as skin irritation reducing agents, analgesics, transdermal absorption enhancers, stabilizers, etc., in addition to the active ingredient on an as needed basis. The mixing method is not particularly limited as long as a W/O emulsion can be formed, and examples of the method include stirring by a homogenizer or the like. The stirring by a homogenizer may be performed under conditions, for example, at about 5,000 to 50,000 rpm, more preferably about 10,000 to 30,000 rpm.

The weight ratio between the active ingredient and the surfactant (active ingredient weight/surfactant weight) in the W/O emulsion is not particularly limited as long as the particle of the present invention can be eventually obtained, and may be, for example, 1:3 to 1:100, preferably 1:5 to 1:70, still more preferably 1:10 to 1:50.

The method for drying a W/O emulsion containing an active ingredient in the aqueous phase is not particularly limited as long as the solvents (aqueous solvent and oily solvent) in the emulsion can be removed, and examples thereof include freeze drying and vacuum drying. In particular, freeze drying is preferred.

The particle after drying may be directly mixed with the base phase, or may be mixed with a base or the like

once and then mixed with the base phase.

3.2 Method for preparing base phase

The method for preparing a base phase of the present invention described in the section 2 comprises at least a step (I) of mixing a particle including a first fraction containing an active ingredient and a second fraction containing a surfactant with one or more bases, and a step of gelling the one or more bases before or after the step (I). More specifically, a first method and a second method described below are included.

(1) First method

A first method for preparing a base phase comprises a step (1) of preparing a base containing a gelatinous liquid component described in the section 2.1, and a step (2) of preparing a base phase by mixing a particle comprising a first fraction containing an active ingredient and a second fraction containing a surfactant with the base containing a gelatinous liquid component.

The step (1) may be the step of preparing a gelatinous liquid component, or may be the step of providing a prepared gelatinous liquid component. The method of preparing a gelatinous liquid component may include the steps of, for example, mixing a liquid agent with a gelling agent according to the component ratio described in the section 2, dissolving both of the components by heating at about 50 to 130°C for about 0.5 to 5 hours, and then curing the product at 20 to 40°C for about 1 to 24 hours.

In the subsequent step (2), the gelatinous liquid component prepared in the step (1), the other base described in the section 2. 1, and if necessary, an adhesive, a tackifier, and other components are mixed. The mixture thus prepared may be further mixed with the particle described in the section 1, so as to prepare a base phase. The mixing method is not particularly limited, and a commonly known method may be applied. For example, the agitation may be performed by a vacuum mixer at 2000 rpm, for 2 minutes, under less than 1 Pa.

In another aspect of the step (2), the particle, the gelatinous liquid component prepared in the step (1), the other base described in the section 2., and if necessary, an adhesive, a tackifier, and other components are mixed in a solvent such as cyclohexane, hexane, toluene and ethyl acetate to form a mixture (hereinafter, referred to as base phase solution 1), and the solvent is then removed to prepare the base phase.

Furthermore, in an additional step (3), the whole of the base phase may be gelled by heating the base phase prepared in the step (2) once to about 50 to 100°C for elimination of the cross-linking of the gel, and then adding a gelling agent or heating the base phase for the second time. The additional step (3) is particularly preferably applied to a gelatinous liquid component prepared by physical cross-linking.

(2) Second method

A second method for preparing a base phase includes the steps of mixing the particle described in the section 1, the base described in the section 2, and if necessary, an adhesive, a tackifier, and other components, and gelling the whole of the base phase by adding a gelling agent or by heat treatment. In another aspect, the particle described in the section 1, the base described in the section 2, a gelling agent, and if necessary, an adhesive, a tackifier, and other components are mixed in a solvent such as cyclohexane, hexane, toluene and ethyl acetate to form a mixture (hereinafter, referred to as base phase solution 2), which is then gelled by removing the solvent before completion of the external preparation. The liquid agent may be gelled by heating at about 50 to 130°C, for about 0.5 to 5 hours.

3.3 Method for preparing external preparation

(1) Patch

External preparations for use in patches may be produced, for example, by a solution coating method. In the solution coating method, the base phase solution 1 or the base phase solution 2 prepared as described above may be used. The concentration of the solid content in each of the base phase solutions is preferably 10 to 80 wt%, more preferably 20 to 60 wt%.

Subsequently, each of the base phase solutions is uniformly applied onto a release liner (e.g., silicone treated polyester film having resistance to organic solvent) with an applicator such as a knife coater, a comma coater and a reverse coater, and dried to remove the solvent for completion of a base phase layer, on which a substrate (e.g., polyester film or nonwoven fabric having resistance to organic solvent) is laminated, so that a patch can be obtained. Depending on the type of substrate, after formation of a base phase layer on a substrate, a release layer may be laminated on the surface of the base phase.

In an another method, for example, the base phase may be held on a synthetic fiber fabric member of polyester, polyethylene, or the like, or on a woven or non-woven fabric made from an appropriate combination thereof, or a permeable membrane by lamination, impregnation or the like, and further covered with an adhesive cover material or the like for use.
The patches thus obtained are appropriately cut into a shape such as ellipse, a circle, a square, and a rectangle, depending on the intended use. An adhesive layer may be provided in the periphery on an as needed basis.

(2) Ointment

An oily ointment can be prepared, for example, by a known method described in the Manual of Japanese Pharmacopoeia or the like. For example, first, the oily base is heated to about 70 to 80°C to make an oil phase (solution state). Separately, the particle is dissolved or dispersed in a base or the like to prepare a principal agent phase. The oil phase and the principal agent phase are mixed and cooled to obtain an oily ointment. On this occasion, the gelatinous liquid component can be added to the oil phase or the principal agent phase. Alternatively, after mixing of the oil phase and the principal agent phase, a gelling agent may be added.

(3) External preparation in other dosage form

External preparations in other dosage forms can be also prepared, for example, by a known method described in the Manual of Japanese Pharmacopoeia or the like. As described in the method for preparing a base phase, a base phase may be completed by gelling the liquid agent contained in the base phase in advance, or by adding a gelling agent after mixing all the components of the base phase.

Examples

The present invention is described in detail with reference to Examples and Test Examples as follows, though the present invention is not limited thereto.

Reference Preparation Example: Preparation of the particle

A particle comprising a first fraction containing an active ingredient and a second fraction containing a surfactant were prepared by the following preparation method.
In 40 g of pure water, 0.1 g of donepezil hydrochloride as an active ingredient was dissolved. A solution of 1.5 g of sucrose erucic acid ester as surfactant (manufactured by Mitsubishi-Chemical Foods Corporation, ER-290, containing diester and triester as main components) dissolved in 80 g of cyclohexane was added to the mixture, which was stirred with a homogenizer (10,000 rpm). The solution was then freeze-dried for 2 days to obtain a particle.

Preparation Examples 1 to 6: Preparation of gelatinous liquid component

A gelatinous liquid component was prepared by the following method.
According to the ratio shown in Table 1, a liquid paraffin (manufactured by Wako Pure Chemical Industries, Ltd., density: 0.800 to 0.835 g/mL) was mixed with dextrin palmitate (manufactured by Chiba Flour Milling Co., Ltd., Rheopearl KL2). During mixing, a predetermined amount of dextrin palmitate was slowly added to the liquid paraffin while stirring with a stirrer. Stirring was then performed at 120°C for 2 hours, so that the dextrin palmitate was dissolved. The solution was then allowed to stand at 40°C for 16 hours, so that a gelatinous liquid paraffin was prepared.
In the same manner as in gelling of the liquid paraffin, isopropyl myristate (IPM) was also mixed with dextrin palmitate according to the ratio shown in Table 2, so that a gelatinous isopropyl myristate was obtained.

[Table 1]

| Preparation Example | Liquid paraffin (wt%) | Dextrin palmitate (wt%) |
|---|---|---|
| 1 | 97.5 | 2.5 |
| 2 | 95.0 | 5.0 |
| 3 | 92.5 | 7.5 |

[Table 2]

| Preparation Example | IPM (wt%) | Dextrin palmitate (wt%) |
|---|---|---|
| 4 | 97.5 | 2.5 |
| 5 | 95.0 | 5.0 |
| 6 | 92.5 | 7.5 |

Example 1

To 30 parts by weight of a particle obtained in the Reference Preparation Example, 20 parts by weight of a styrene-isoprene-styrene block copolymer as rubber adhesive (SIS, manufactured by Zeon Corporation, QUINTAC 3520), 20 parts by weight of a tackifier (alicyclic saturated hydrocarbon resin, manufactured by Arakawa Chemical Industries, Ltd., ARKON P100), 20 parts by weight of the gelatinous liquid paraffin prepared in the Preparation Example 1, and 10 parts by weight of ungelled isopropyl myristate were blended. To the mixture, cyclohexane was added to give a concentration of solid content of 30 wt%, and mixed until a uniform state was obtained. A base phase solution was thus prepared.

Subsequently, silicone was applied to one surface of a release substrate made of polyethylene terephthalate film having a thickness of 38 μm to prepare a release-treated release sheet functioning as release liner. The base phase solution was applied to a release-treated surface of the release sheet, and dried at 60°C for 60 minutes, so that a laminate having a base phase layer on the release-treated surface of the release sheet was manufactured. Subsequently, a substrate made of polyethylene terephthalate film having a thickness of 38 μm was arranged.

In producing a patch, one surface of the substrate and the base phase layer of the laminate were layered face to face, so that the base phase layer of the laminate was transferred to the substrate to obtain a unified laminate.

Example 2

To 30 parts by weight of the particle obtained in the Reference Preparation Example, 20 parts by weight of a styrene-isoprene-styrene block copolymer, 20 parts by weight of a tackifier, 20 parts by weight of the gelatinous liquid paraffin prepared in the Preparation Example 2, and 10 parts by weight of ungelled isopropyl myristate were blended. To the mixture, cyclohexane was added to give a concentration of solid content of 30 wt%, and mixed until a uniform state was obtained. A base phase solution was thus prepared. Subsequently, a patch was prepared in the same manner as in Example 1.

Example 3

To 30 parts by weight of the particle obtained in the Reference Preparation Example, 20 parts by weight of a styrene-isoprene-styrene block copolymer, 20 parts by weight of a tackifier, 20 parts by weight of the gelatinous liquid paraffin prepared in the Preparation Example 3, and 10 parts by weight of ungelled isopropyl myristate were blended. To the mixture, cyclohexane was added to give a concentration of solid content of 30 wt%, and mixed until a uniform state was obtained. A base phase solution was thus prepared. Subsequently, a patch was produced in the same manner as in Example 1.

Example 4

To 30 parts by weight of the particle obtained in the Reference Preparation Example, 20 parts by weight of a styrene-isoprene-styrene block copolymer, 20 parts by weight of a tackifier, 20 parts by weight of the gelatinous liquid paraffin prepared in the Preparation Example 2, and 10 parts by weight of gelled isopropyl myristate prepared in the Preparation Example 4 were blended. To the mixture, cyclohexane was added to give a concentration of solid content of 30 wt%, and mixed until a uniform state was obtained. A base phase solution was thus prepared. Subsequently, a patch was prepared in the same manner as in Example 1.

Example 5

To 30 parts by weight of the particle obtained in the Reference Preparation Example, 20 parts by weight of a styrene-isoprene-styrene block copolymer, 20 parts by weight of a tackifier, 20 parts by weight of the gelatinous liquid paraffin prepared in the Preparation Example 2, and 10 parts by weight of gelled isopropyl myristate prepared in the

Preparation Example 5 were blended. To the mixture, cyclohexane was added to give a concentration of solid content of 30 wt%, and mixed until a uniform state was obtained. A base phase solution was thus prepared. Subsequently, a patch was produced in the same manner as in Example 1.

Example 6

To 30 parts by weight of the particle obtained in the Reference Preparation Example, 20 parts by weight of a styrene-isoprene-styrene block copolymer, 20 parts by weight of a tackifier, 20 parts by weight of the gelatinous liquid paraffin prepared in the Preparation Example 2, and 10 parts by weight of gelled isopropyl myristate prepared in the Preparation Example 6 were blended. To the mixture, cyclohexane was added to give a concentration of solid content of 30 wt%, and mixed until a uniform state was obtained. A base phase solution was thus prepared. Subsequently, a patch was produced in the same manner as in Example 1.

Comparative Example 1

To 30 parts by weight of the particle obtained in the Reference Preparation Example, 20 parts by weight of a styrene-isoprene-styrene block copolymer, 20 parts by weight of a tackifier, 20 parts by weight of the ungelled liquid paraffin, and 10 parts by weight of ungelled isopropyl myristate were blended. To the mixture, cyclohexane was added to give a concentration of solid content of 30 wt%, and mixed until a uniform state was obtained. A base phase solution was thus prepared. Subsequently, a patch was produced in the same manner as in Example 1.

Test for confirming bleed-out phenomenon

After storage of the patches prepared in Examples 1 to 6 and Comparative Example 1 at 40°C for 3 days, the release sheet was detached and subjected to visual inspection for the presence of a particle adhering to the release sheet.

The results are shown in Table 3.

[Table 3]

<table>
<tr><th></th><th>Particle</th><th>Adhesive</th><th>Tackifier</th><th colspan="2">Liquid paraffin</th><th colspan="2">IPM</th><th>Occurrence of bleed-out phenomenon</th></tr>
<tr><td>Comp. Ex. 1</td><td rowspan="7">30</td><td rowspan="7">20</td><td rowspan="7">20</td><td rowspan="7">20</td><td>Ungelled</td><td rowspan="7">10</td><td>Ungelled</td><td>Present</td></tr>
<tr><td>Ex. 1</td><td>Preparation Example 1</td><td>Ungelled</td><td>Absent</td></tr>
<tr><td>Ex. 2</td><td>Preparation Example 2</td><td>Ungelled</td><td>Absent</td></tr>
<tr><td>Ex. 3</td><td>Preparation Example 3</td><td>Ungelled</td><td>Absent</td></tr>
<tr><td>Ex. 4</td><td>Preparation Example 2</td><td>Preparation Example 4</td><td>Absent</td></tr>
<tr><td>Ex. 5</td><td>Preparation Example 2</td><td>Preparation Example 5</td><td>Absent</td></tr>
<tr><td>Ex. 6</td><td>Preparation Example 2</td><td>Preparation Example 6</td><td>Absent</td></tr>
<tr><td colspan="9">* The figures in the table represent the content (wt%) in the base phase.</td></tr>
</table>

As shown in Table 3, when both of the liquid paraffin and isopropyl myristate were ungelled for use, the presence of a particle adhering to the release sheet was confirmed, which revealed the occurrence of bleed-out phenomenon. In contrast, it was revealed that the bleed-out phenomenon can be prevented by gelling the liquid paraffin or both of the liquid paraffin and isopropyl myristate.

Reference Preparation Example 2: Preparation of a particle

A particle comprising a first fraction containing an active ingredient and a second fraction containing a surfactant was prepared by the following preparation method.

In 40 g of pure water, 0.1 g of hemipenta hydrate risedronate monosodium (manufactured by Tokyo Chemical Industry Co., Ltd., molecular weight: 306, octanol/water partition coefficient: -5.0) as an active ingredient was dissolved. A solution of 1.5 g of glyceryl caprylate (manufactured by Taiyo Kagaku Co., Ltd., SUNSOFT No. 707-C, containing the monoester and diester as main components) as surfactant dissolved in 80 g of cyclohexane was added thereto, and the mixture was stirred with a homogenizer (10,000 rpm). The solution was then freeze-dried for 2 days to obtain a particle.

Preparation Examples 7 to 9: Preparation of gelatinous liquid component

Gelatinous liquid components were prepared by the following preparation method.

According to the ratio shown in Table 4, a liquid paraffin (manufactured by Wako Pure Chemical Industries, Ltd., density: 0.800 to 0.835 g/mL) was mixed with dextrin palmitate (manufactured by Chiba Flour Milling Co., Ltd., Rheopearl KL2). During mixing, a predetermined amount of dextrin palmitate was slowly added to the liquid paraffin while stirring with a stirrer. Stirring was then performed at 120°C for 2 hours, so that the dextrin palmitate was dissolved. The solution was then allowed to stand at 40°C for 16 hours, so that a gelatinous liquid paraffin was prepared.

[Table 4]

| Preparation Example | Liquid paraffin (wt%) | Dextrin palmitate (wt%) |
|---|---|---|
| 7 | 95 | 5 |
| 8 | 90 | 10 |
| 9 | 85 | 15 |

Preparation Examples 10 to 12: Preparation of gelatinous liquid component

According to the ratio shown in Table 5, a liquid paraffin (manufactured by Wako Pure Chemical Industries, Ltd., density: 0.860 to 0.890 g/mL) was mixed with dextrin (palmitate/ethylhexanoate) (manufactured by Chiba Flour Milling Co., Ltd., Rheopearl TT2). During mixing, a predetermined amount of dextrin (palmitate/ethylhexanoate) was slowly added to the liquid paraffin while stirring with a stirrer. Stirring was then performed at 120°C for 2 hours, so that the dextrin (palmitate/ethylhexanoate) was dissolved. The solution was then allowed to stand at 40°C for 16 hours, so that a gelatinous liquid paraffin was prepared.

Preparation Example 13: Preparation of gelatinous liquid component

According to the ratio shown in Table 6, a liquid paraffin (manufactured by Wako Pure Chemical Industries, Ltd., density: 0.860 to 0.890 g/mL) was mixed with a low-density polyethylene (molecular weight: 21000), so that a gelatinous liquid paraffin was prepared.

[Table 5]

| Preparation Example | Liquid paraffin (wt%) | Dextrin (palmitate/ethylhexanoate) (wt%) |
|---|---|---|
| 10 | 95 | 5 |
| 11 | 90 | 10 |
| 12 | 85 | 15 |

[Table 6]

| Preparation Example | Liquid paraffin (wt%) | Polyethylene (wt%) |
|---|---|---|
| 13 | 95 | 5 |

Example 7

To 40 parts by weight of a particle obtained in the Reference Preparation Example 2, 20 parts by weight of a styrene-isoprene-styrene block copolymer as rubber adhesive (SIS, manufactured by Zeon Corporation, QUINTAC 3520), 20 parts by weight of a tackifier (alicyclic saturated hydrocarbon resin, manufactured by Arakawa Chemical Industries, Ltd., ARKON P100), and 20 parts by weight of the gelatinous liquid paraffin prepared in the Preparation Example 7 were blended. To the mixture, cyclohexane was added to give a concentration of solid content of 30 wt%, and mixed until a uniform state was obtained. A base phase solution was thus prepared. Subsequently, a patch was produced in the same manner as in Example 1.

Example 8

To 40 parts by weight of a particle obtained in the Reference Preparation Example 2, 20 parts by weight of a styrene-isoprene-styrene block copolymer, 20 parts by weight of a tackifier, and 20 parts by weight of the gelatinous liquid paraffin prepared in the Preparation Example 8 were blended. To the mixture, cyclohexane was added to give a concentration of solid content of 30 wt%, and mixed until a uniform state was obtained. A base phase solution was thus prepared. Subsequently, a patch was produced in the same manner as in Example 1.

Example 9

To 40 parts by weight of a particle obtained in the Reference Preparation Example 2, 20 parts by weight of a styrene-isoprene-styrene block copolymer, 20 parts by weight of a tackifier, and 20 parts by weight of the gelatinous liquid paraffin prepared in the Preparation Example 9 were blended. To the mixture, cyclohexane was added to give a concentration of solid content of 30 wt%, and mixed until a uniform state was obtained. A base phase solution was thus prepared. Subsequently, a patch was produced in the same manner as in Example 1.

Example 10

To 30 parts by weight of a particle obtained in the Reference Preparation Example 2, 20 parts by weight of a styrene-isoprene-styrene block copolymer, 20 parts by weight of a tackifier, and 30 parts by weight of the gelatinous liquid paraffin prepared in the Preparation Example 10 were blended. To the mixture, cyclohexane was added to give a concentration of solid content of 30 wt%, and mixed until a uniform state was obtained. A base phase solution was thus prepared. Subsequently, a patch was produced in the same manner as in Example 1.

Example 11

To 30 parts by weight of a particle obtained in the Reference Preparation Example 2, 20 parts by weight of a styrene-isoprene-styrene block copolymer, 20 parts by weight of a tackifier, and 30 parts by weight of the gelatinous liquid paraffin prepared in the Preparation Example 11 were blended. To the mixture, cyclohexane was added to give a concentration of solid content of 30 wt%, and mixed until a uniform state was obtained. A base phase solution was thus prepared. Subsequently, a patch was produced in the same manner as in Example 1.

Example 12

To 30 parts by weight of a particle obtained in the Reference Preparation Example 2, 20 parts by weight of a styrene-isoprene-styrene block copolymer, 20 parts by weight of a tackifier, and 30 parts by weight of the gelatinous liquid paraffin prepared in the Preparation Example 12 were blended. To the mixture, cyclohexane was added to give a concentration of solid content of 30 wt%, and mixed until a uniform state was obtained. A base phase solution was thus prepared. Subsequently, a patch was produced in the same manner as in Example 1.

Example 13

To 30 parts by weight of a particle obtained in the Reference Preparation Example 2, 20 parts by weight of a styrene-isoprene-styrene block copolymer, 20 parts by weight of a tackifier, and 30 parts by weight of the gelatinous liquid paraffin prepared in the Preparation Example 13 were blended. To the mixture, cyclohexane was added to give a concentration of solid content of 30 wt%, and mixed until a uniform state was obtained. A base phase solution was thus prepared. Subsequently, a patch was produced in the same manner as in Example 1.

Example 14

To 30 parts by weight of a particle obtained in the Reference Preparation Example 2, 60 parts by weight of an acrylic adhesive (DURO-TAK 387-2510, manufactured by Henkel AG & Co., solid content: 40 wt%), and 10 parts by weight of the gelatinous isopropyl myristate prepared in the Preparation Example 5 were blended. To the mixture, ethyl acetate was added to give a concentration of solid content of 30 wt%, and mixed until a uniform state was obtained. A base phase solution was thus prepared. Subsequently, a patch was produced in the same manner as in Example 1.

Example 15

To 30 parts by weight of a particle obtained in the Reference Preparation Example 2, 60 parts by weight of an oily ointment base (manufactured by Taisho Pharmaceutical Co., Ltd., PLASTIBASE), and 10 parts by weight of the gelatinous isopropyl myristate prepared in the Preparation Example 5 were blended. The mixture was mixed with a mortar, so that an ointment was produced.

Comparative Example 2

To 30 parts by weight of a particle obtained in the Reference Preparation Example 2, 20 parts by weight of a styrene-isoprene-styrene block copolymer, 20 parts by weight of a tackifier, and 30 parts by weight of the ungelled liquid paraffin were blended. To the mixture, cyclohexane was added to give a concentration of solid content of 30 wt%, and mixed until a uniform state was obtained. A base phase solution was thus prepared. Subsequently, a patch was produced in the same manner as in Example 1.

Comparative Example 3

To 30 parts by weight of a particle obtained in the Reference Preparation Example 2, 60 parts by weight of an acrylic adhesive (DURO-TAK 387-2510, manufactured by Henkel AG & Co., solid content: 40 wt%), and 10 parts by weight of the ungelled liquid paraffin were blended. To the mixture, ethyl acetate was added to give a concentration of solid content of 30 wt%, and mixed until a uniform state was obtained. A base phase solution was thus prepared. Subsequently, a patch was produced in the same manner as in Example 1.

Comparative Example 4

To 30 parts by weight of a particle obtained in the Reference Preparation Example 2, 60 parts by weight of an oily ointment base (manufactured by Taisho Pharmaceutical Co., Ltd., PLASTIBASE), and 10 parts by weight of ungelled isopropyl myristate were blended. The mixture was mixed with a mortar, so that an ointment was produced.

Test for confirming bleed-out phenomenon

After storage of the patches prepared in Examples 7 to 14 and Comparative Examples 2 and 3 at 40°C for 3 days, each of the release sheets was detached and subjected to visual inspection for the presence of a particle adhering to the release sheet. The results are shown in Tables 7 and 8.

Also, after storage of the ointments prepared in Examples 15 and Comparative Example 4 at 60°C for 3 days, the liquid component seepage in a container was visually inspected. The results are shown in Tables 9.

[Table 7]

| | Particle | Adhesive | Tackifier | Liquid paraffin | | Occurrence of bleed-out phenomenon |
|---|---|---|---|---|---|---|
| Comp. Ex. 2 | 30 | 20 | 20 | 30 | Ungelled | Present |

(continued)

| | Particle | Adhesive | Tackifier | Liquid paraffin | | Occurrence of bleed-out phenomenon |
|---|---|---|---|---|---|---|
| Ex. 7 | 40 | 20 | 20 | 20 | Preparation Example 7 | Absent |
| Ex. 8 | | | | | Preparation Example 8 | Absent |
| Ex. 9 | | | | | Preparation Example 9 | Absent |
| Ex. 10 | 30 | | | 30 | Preparation Example 10 | Absent |
| Ex. 11 | | | | | Preparation Example 11 | Absent |
| Ex. 12 | | | | | Preparation Example 12 | Absent |
| Ex. 13 | | | | | Preparation Example 13 | Absent |
| The figures in the table represent the content (wt%) in the base phase. | | | | | | |

[Table 8]

| | Particle | Acrylic adhesive | IPM | | Occurrence of bleed-out phenomenon |
|---|---|---|---|---|---|
| Comp. Ex. 3 | 30 | 60 | 10 | Ungelled | Present |
| Ex. 14 | 30 | 60 | 10 | Preparation Example 5 | Absent |
| * The figures in the table represent the content (wt%) in the base phase. | | | | | |

[Table 9]

| | Particle | PLASTIBASE ointment | IPM | | Occurrence of bleed-out phenomenon |
|---|---|---|---|---|---|
| Comp. Ex. 4 | 30 | 60 | 10 | Ungelled | Present |
| Ex. 15 | 30 | 60 | 10 | Preparation Example 5 | Absent |
| * The figures in the table represent the content (wt%) in the base phase. | | | | | |

As shown in Tables 7 to 9, it was revealed that the bleed-out phenomenon can be prevented by gelling of liquid paraffin and isopropyl myristate (IPM).

## Claims

1. An external preparation comprising:

   a particle including a first fraction containing an active ingredient and a second fraction containing a surfactant; and
   a base phase containing a gelatinous liquid component.

2. The external preparation according to claim 1, wherein the particle includes a part or the whole of the surface of the first fraction covered with the second fraction.

3. The external preparation according to claim 1 or 2, wherein the gelatinous liquid component content is in the range of 1 to 50 wt% relative to 100 wt% of the base phase.

4. The external preparation according to any one of claims 1 to 3, wherein the gelatinous liquid component contains a liquid agent and a gelling agent, with a gelling agent content in the range of 1 to 30 wt% relative to 100 wt% of the liquid agent.

5. The external preparation according to claim 4, wherein the liquid agent is at least one oily base selected from the

group consisting of hydrocarbons, alcohol carboxylic acid esters, polyalcohol fatty acid esters, and oxy acid esters.

**6.** The external preparation according to claim 4 or 5, wherein the gelling agent is at least one selected from the group consisting of polyethylene, dextrin palmitate, dextrin (palmitate/ethylhexanoate), dextrin myristate, and inulin stearate.

**7.** The external preparation according to any one of claims 1 to 6, wherein the active ingredient is hydrophilic.

**8.** The external preparation according to any one of claims 1 to 7, wherein the external preparation is a patch or an ointment.

**9.** The external preparation according to claim 8, wherein the patch contains an adhesive in the base phase.

**10.** The external preparation according to claim 9, wherein the adhesive is a rubber adhesive or an acrylic adhesive.

**11.** A method for producing an external preparation comprising a step (I) of mixing a particle including a first fraction containing an active ingredient and a second fraction containing a surfactant with one or more bases, and a step of preparing a base phase by gelling the one or more bases before or after the step (I).

**12.** A method for producing an external preparation comprising:

a step (1) of preparing a base containing a gelatinous liquid component; and
a step (2) of preparing a base phase by mixing a particle including a first fraction containing an active ingredient and a second fraction containing a surfactant with the base containing a gelatinous liquid component.

**13.** The method for producing an external preparation according to claim 12, wherein the step (1) comprises mixing a liquid agent with a gelling agent.

**14.** The method for producing an external preparation according to claim 13, wherein the liquid agent is an oily base.

## INTERNATIONAL SEARCH REPORT

International application No. PCT/JP2016/089052

A. CLASSIFICATION OF SUBJECT MATTER

*A61K9/70*(2006.01)i, *A61K9/06*(2006.01)i, *A61K9/16*(2006.01)i, *A61K31/445*(2006.01)i, *A61K47/06*(2006.01)i, *A61K47/14*(2006.01)i, *A61K47/32*(2006.01)i, *A61K47/36*(2006.01)i

According to International Patent Classification (IPC) or to both national classification and IPC

B. FIELDS SEARCHED

Minimum documentation searched (classification system followed by classification symbols)

A61K9/70, A61K9/06, A61K9/16, A61K31/445, A61K47/06, A61K47/14, A61K47/32, A61K47/36

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)

CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| Y | WO 2011/059037 A1 (Nihon University), 19 May 2011 (19.05.2011), claims 1 to 46; paragraphs [0023], [0027]; examples 1 to 10 & US 2012/0264742 A1 claims 1 to 46; paragraphs [0034], [0038]; examples 1 to 10 & EP 2500038 A1 & KR 10-2012-0089336 A & CN 102781473 A | 1-14 |
| Y | JP 2010-163398 A (Hisamitsu Pharmaceutical Co., Inc.), 29 July 2010 (29.07.2010), claims 1 to 3; paragraphs [0019], [0035], [0037]; example 1 (Family: none) | 1-14 |

☒ Further documents are listed in the continuation of Box C.  ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 01 February 2017 (01.02.17) | 14 February 2017 (14.02.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2016/089052

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2006/025583 A1 (ASPION Co., Ltd.), 09 March 2006 (09.03.2006), claims 1 to 20; test examples 1, 2 & US 2009/0238846 A1 claims 1 to 20; test examples 1, 2 & EP 1785131 A1 & CN 101022785 A | 1-14 |
| A | JP 1-203319 A (Taisho Pharmaceutical Co., Ltd.), 16 August 1989 (16.08.1989), claims 1, 2 (Family: none) | 1-14 |
| A | JP 2014-172840 A (Lintec Corp.), 22 September 2014 (22.09.2014), claims 1 to 8; examples 1, 2 (Family: none) | 1-14 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2004075540 A
- JP 2013227288 A
- JP 2015145429 A
- JP 2014172840 A

### Non-patent literature cited in the description


- **TOSHINAO OKITSU.** *Journal of the Adhesion Society of Japan,* 1993, vol. 29 (5), 204-211